# EUROPEAN PATENT APPLICATION

(11) **EP 4 555 928 A1**
(43) Date of publication of application: **21.05.2025**
(21) Application number: 24213191.0
(22) Date of filing: 15.11.2024
(51) Int. Cl.: A61B 5/145, A61B 5/1495, A61B 5/00, G16H 40/40, G16H 50/20

(54) **SYSTEMS AND METHODS FOR REDUCING SENSOR VARIABILITY**

(30) Priority: 16.11.2023 WO PCT/GR2023/000061; 01.11.2024 US 202418934534
(71) Applicant: Medtronic MiniMed, Inc., Northridge, CA 91325 (US)
(72) Inventor: ARGUELLES MORALES, Juan Enrique, Northridge, 91325 (US); SELEH, Sadaf Soleymani, Northridge, 91325 (US); NAVA-GUERRA, Leonardo, Northridge, 91325 (US); NOTGHI, Bahram B., Northridge, 91325 (US); MALLAS, Georgios, Northridge, 91325 (US); PICCININI, Francesca, Northridge, 91325 (US); AROYAN, Sarkis D., Northridge, 91325 (US); GARAI, Ellis, Northridge, 91325 (US); SHAH, Zachary Andrew, Northridge, 91325 (US)
(74) Representative: Zimmermann & Partner Patentanwälte mbB

(57) **Abstract**

A system for reducing sensor variability includes a sensor configured to generate real-time data relating to glucose sensitivity. The system causes performance of accessing the real-time data from the sensor relating to glucose sensitivity and inputting the real-time data into a machine learning model. The system also causes performance of estimating by the machine learning model an expected glucose sensitivity based on the real-time data and correcting the glucose sensitivity based on the expected glucose sensitivity.

## Description

### FIELD

The present disclosure relates generally to continuous glucose monitoring (CGM) and more particularly to systems and methods for improving run-in times for sensors used in CGM.

### BACKGROUND

Analyte sensors such as biosensors include devices that use biological elements to convert a chemical analyte in a matrix into a detectable signal. There are many types of biosensors used for a wide variety of analytes, including amperometric glucose sensors for glucose level control for diabetes.

A typical glucose sensor works according to the following chemical reactions: 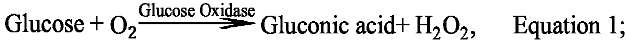 and 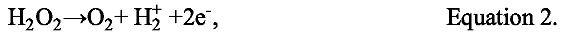 The glucose oxidase is used to catalyze the reaction between glucose and oxygen to yield gluconic acid and hydrogen peroxide, H₂O₂, (Equation 1). The hydrogen peroxide reacts electrochemically as shown in Equation 2, and the current can be measured by a potentiostat. These reactions, which occur in a variety of oxidoreductases known in the art, are used in a number of sensor designs.

One common problem with analyte sensors is that they can electrochemically react not only with the analyte to be measured (or by-product of the enzymatic reaction with the analyte), but can also react with other electroactive chemical species that are not intentionally being measured, which causes an increase in signal strength due to these "interfering species." Typically, such interfering species are compounds with an oxidation or reduction potential that overlaps with the analyte to be measured (or a by-product of the enzymatic reaction with the analyte). For example, in a conventional amperometric glucose oxidase-based glucose sensor where the conventional sensor measures hydrogen peroxide, interfering species such as acetaminophen, ascorbate, and urate are known to confound true analyte signals, resulting in loss of sensor sensitivity or longevity.

Another common problem is that the analyte sensors need time for sensor signals to stabilize due to different sensor designs. The term "run-in" is typically used to describe the period of time needed by the sensor to stabilize during the first few days of testing/wearing of the sensor. There is room for improvement in the design of analyte sensors to reduce sensor run-in time and/or improve sensor lifetime.

### SUMMARY

The present disclosure relates to systems and methods for improving sensor sensitivity for sensors used in continuous glucose monitoring.

In accordance with aspects of the present disclosure, a system for reducing sensor variability, includes a sensor configured to generate real-time data relating to glucose sensitivity, one or more processors, and one or more processor-readable media storing instructions which, when executed by the one or more processors, causes performance of accessing the real-time data from the sensor relating to glucose sensitivity; inputting the real-time data into a machine learning model; estimating by the machine learning model an expected glucose sensitivity based on the real-time data; and correcting the glucose sensitivity based on the expected glucose sensitivity.

In an aspect of the present disclosure, the real-time data may include electrical properties associated with the sensor.

In another aspect of the present disclosure, the real-time data may include an Isig value and at least one of a Vcntr value or an EIS signature value.

In yet another aspect of the present disclosure, estimating by the machine learning model the corrected glucose sensitivity may include estimating an Isig trend over time for the sensor.

In a further aspect of the present disclosure, the Isig trend over time may be independent of any sensed glucose fluctuations.

In yet a further aspect of the present disclosure, a calibration ratio may be a blood glucose value divided by Isig. The instructions, when executed by the one or more processors, may further cause performance of stabilizing the calibration ratio based on correcting Isig using the estimated Isig trend over time and the EIS signature value.

In an aspect of the present disclosure, the real-time data includes a portion that may be based on a blood glucose value and a portion that is unrelated to the blood glucose value. The instructions, when executed by the processor, may further cause the system to determine, by the machine learning model, a portion of the real-time data that is unrelated to the blood glucose value.

In another aspect of the present disclosure, the instructions, when executed by the one or more processors, may further cause performance of generating an adjusted Isig based on the determined portion of the real-time data that is unrelated to the blood glucose value.

In yet another aspect of the present disclosure, when correcting the glucose sensitivity, the instructions, when executed by the one or more processors, may further cause performance of estimating by the machine learning model a calibration factor as a function of time based on the real-time data; and calibrating the real-time data at least in part based on the calibration factor.

In a further aspect of the present disclosure, the portion of the real-time data that is unrelated to the blood glucose value may be based on a design configuration of the sensor.

In accordance with aspects of the disclosure, a processor-implemented method of correcting real-time sensor data is presented. The method includes: accessing real-time data from a sensor relating to glucose sensitivity; inputting the real-time data into a machine learning model; estimating by the machine learning model an expected glucose sensitivity based on the input real-time data; and correcting the glucose sensitivity based on the expected glucose sensitivity.

In yet a further aspect of the present disclosure, the real-time data may include sensor electrical properties associated with the sensor.

In an aspect of the present disclosure, the real-time data may include an Isig value and at least one of a Vcntr value or an EIS signature value. In another aspect of the present disclosure, estimating by the machine learning model the compensated glucose sensitivity may include estimating an Isig trend over time for the sensor.

In yet another aspect of the present disclosure, the Isig trend over time may be independent of any sensed glucose fluctuations.

In a further aspect of the present disclosure, a calibration ratio may be a blood glucose value divided by Isig. The method may further include stabilizing the calibration ratio over time based on correcting Isig using the estimated Isig trend over time.

In yet a further aspect of the present disclosure, the real-time data may include a portion that is based on a blood glucose value and a portion that is unrelated to the blood glucose value. The method may further include determining, by the machine learning model, a portion of the real-time data that is unrelated to the blood glucose value.

In an aspect of the present disclosure, the method may further include generating an adjusted Isig based on the determined portion of the real-time data that is unrelated to the blood glucose value.

In another aspect of the present disclosure, when correcting the glucose sensitivity, the method may further include estimating by the machine learning model a calibration factor as a function of time based on the input real-time data and calibrating the real-time data at least in part based on the calibration factor.

In accordance with aspects of the disclosure, one or more non-transitory processor readable media storing instructions which, when executed by one or more processors, cause performance of accessing real-time data from a sensor relating to glucose sensitivity; inputting the real-time data into a machine learning model; estimating by the machine learning model an expected glucose sensitivity based on the input real-time data; and correcting the glucose sensitivity based on the expected glucose sensitivity.

Further disclosed herein is a system for reducing sensor variability which includes a sensor configured to generate real-time data relating to glucose sensitivity. The system causes performance of accessing the real-time data from the sensor relating to glucose sensitivity and inputting the real-time data into a machine learning model. The system also causes performance of estimating by the machine learning model an expected glucose sensitivity based on the real-time data and correcting the glucose sensitivity based on the expected glucose sensitivity.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

A detailed description of aspects of the disclosure will be made with reference to the accompanying drawings, wherein like numerals designate corresponding parts in the figures.
FIG. 1 illustrates a perspective view of a subcutaneous sensor insertion set and a block diagram of an analyte sensor electronics device, in accordance with one or more aspects;
FIG. 2 illustrates a substrate having a first side which contains an electrode configuration and a second side which contains electronic circuitry, in accordance with one or more aspects;
FIG. 3 illustrates a block diagram of an electronic circuit for sensing an output of an analyte sensor, in accordance with one or more aspects;
FIG. 4 illustrates a block diagram of an analyte sensor electronics device and a sensor including a plurality of electrodes, in accordance with one or more aspects;
FIG. 5 illustrates an alternative aspect including a sensor and a sensor electronics device, in accordance with one or more aspects;
FIG. 6 is a block diagram of a machine learning network with inputs and outputs of a deep learning neural network, in accordance with aspects of the disclosure;
FIG. 7 is a diagram of layers of the machine learning network of FIG. 6, in accordance with aspects of the disclosure;
FIG. 8 illustrates a flowchart of an exemplary method of adjusting a sensor value when an early wear run-in problem occurs in an analyte sensor, in accordance with aspects of the disclosure;
FIG. 9 illustrates a flowchart of an exemplary method of adjusting a sensor value when an early wear run-in problem occurs in an analyte sensor, in accordance with one or more aspects;
FIG. 10 is a graph the illustrates day 1 run-in for an implanted sensor, in accordance with one or more aspects;
FIG. 11 illustrates example measured Isig values vs expected Isig values over time, in accordance with one or more aspects;
FIG. 12 is a graph illustrating example Isig mean and Isig standard deviation over time, in accordance with one or more aspects;
FIG. 13 is a graph illustrating an example Isig mean vs Isig standard deviation, in accordance with one or more aspects;
FIG. 14 is a graph illustrating an example of Isig and blood glucose (BG) over time, in accordance with one or more aspects;
FIG. 15 is a graph illustrating an example of adjusted Isig and blood glucose (BG) over time. In this graph, Isig has been adjusted using the disclosed methods, in accordance with one or more aspects;
FIG. 16 is a graph illustrating an example of adjusted Isig (Isig_{corrected}) expected Isig values over time over time, in accordance with one or more aspects;
FIG. 17 is a graph illustrating an example of a normalized c for both the original measured Isig and the adjusted Isig, in accordance with one or more aspects; and
FIG. 18 is a graph illustrating example raw Isig values, BG, and adjusted Isig values, in accordance with one or more aspects.

### DETAILED DESCRIPTION

In the following description, reference is made to the accompanying drawings which form a part hereof and which illustrate several aspects of the present disclosure. It is understood that other aspects may be utilized, and structural and operational changes may be made without departing from the scope of the present disclosure.

The aspects herein are described below with reference to flowchart illustrations of methods, systems, devices, apparatus, and programming and computer program products. It will be understood that each block of the flowchart illustrations, and combinations of blocks in the flowchart illustrations, can be implemented by programming instructions, including computer program instructions (as can any menu screens described in the figures). These computer program instructions may be loaded onto a computer or other programmable data processing apparatus (such as a controller, microcontroller, or processor in a sensor electronics device) to produce a machine, such that the instructions which execute on the computer or other programmable data processing apparatus create instructions for implementing the functions specified in the flowchart block or blocks. These computer program instructions may also be stored in a computer-readable memory that can direct a computer or other programmable data processing apparatus to function in a particular manner, such that the instructions stored in the computer-readable memory produce an article of manufacture including instructions which implement the function specified in the flowchart block or blocks. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions specified in the flowchart block or blocks, and/or menus presented herein. Programming instructions may also be stored in and/or implemented via electronic circuitry, including integrated circuits (ICs) and Application Specific Integrated Circuits (ASICs) used in conjunction with sensor devices, apparatuses, and systems.

Long run-in continuous glucose monitoring (CGM) times represent a challenge. The disclosed technology leverages continuous sensor impedance measurements (e.g., EIS) to stabilize sensor current (Isig) over time. The proportionality between the two signals is processed using machine learning to normalize Isig and narrow down signal distribution across sensors, reduce analyte sensor variability, and reduce sensor performance error. The disclosed technology provides the benefit of being agnostic of any physical interpretation of these signals. The disclosed technology further provides the benefit of improving CGM accuracy that can help achieve non-adjunctive labeling for day 1 and provide better analyte sensor accuracy and potential to meet integrated continuous glucose monitoring (iCGM) on day 1. The term "day 1" as used herein includes the first day of use of the analyte sensor after the analyte sensor is implanted in a user. The disclosed technology leverages EIS signals to stabilize sensor sensitivity, in any period of sensor wear where the sensitivity of the sensor varies, including towards the end of sensor wear, where sensitivity loss can lead to early termination.

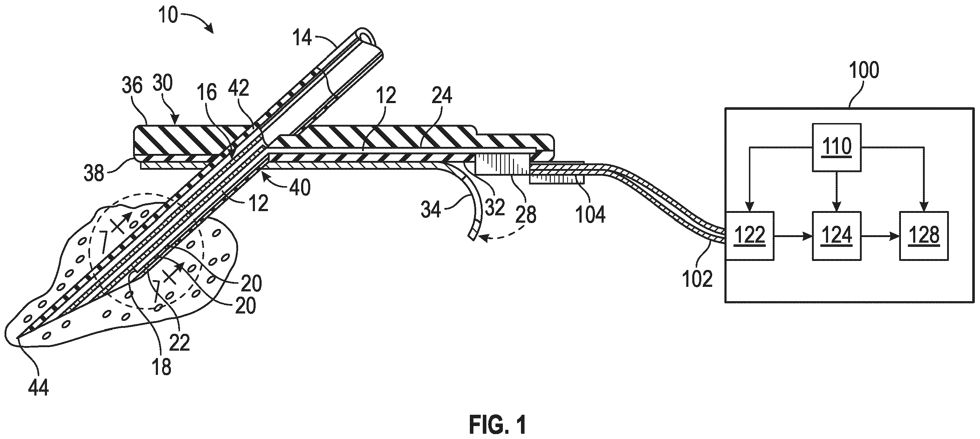

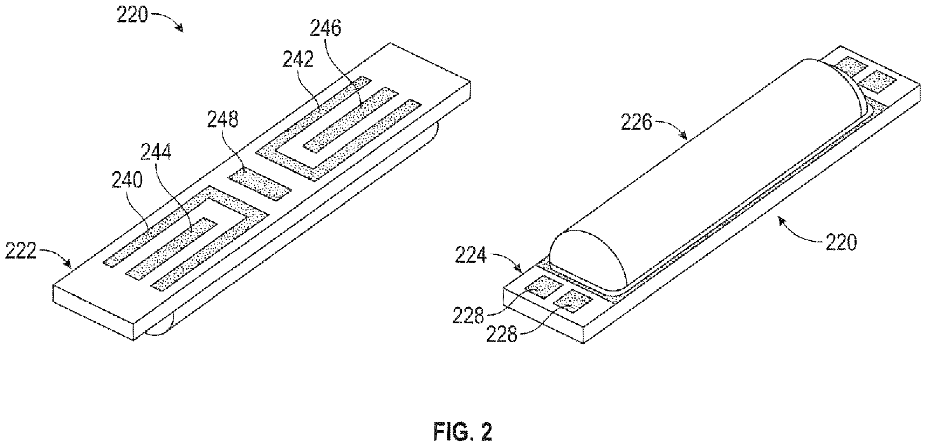

FIG. 1 is a perspective view of a subcutaneous sensor insertion set and a block diagram of a sensor electronics device according to various aspects of the disclosure. As illustrated in FIG. 1, a subcutaneous sensor set 10 is provided for subcutaneous placement of an active portion of an analyte sensor 12 (see, e.g., FIG. 2), or the like, at a selected site in the body of a user. The subcutaneous or percutaneous portion of the sensor set 10 includes a hollow, slotted insertion needle 14, and a cannula 16. The needle 14 is used to facilitate quick and easy subcutaneous placement of the cannula 16 at the subcutaneous insertion site. Inside the cannula 16 is a sensing portion 18 of the analyte sensor 12 to expose one or more sensor electrodes 20 to the user's bodily fluids through a window 22 formed in the cannula 16. In an aspect of the disclosure, the one or more sensor electrodes 20 may include a counter electrode, a reference electrode, and one or more working electrodes. After insertion, the insertion needle 14 is withdrawn to leave the cannula 16 with the sensing portion 18 and the sensor electrodes 20 in place at the selected insertion site.

In particular aspects, the subcutaneous sensor set 10 facilitates accurate placement of a flexible thin film electrochemical analyte sensor 12 of the type used for monitoring specific blood parameters representative of a user's condition. The analyte sensor 12 monitors glucose levels in the body and may be used in conjunction with automated or semi-automated medication infusion pumps of the external or implantable type as described, e.g., in U.S. Patent Nos. 4,562,751; 4,678,408; 4,685,903 or 4,573,994, the entire contents of which are incorporated herein by reference, to control delivery of insulin to a diabetic patient.

Particular aspects of the flexible analyte sensor 12 are constructed in accordance with thin film mask techniques to include elongated thin film conductors embedded or encased between layers of a selected insulative material such as polyimide film or sheet, and membranes. The sensor electrodes 20 at a tip end of the sensing portion 18 are exposed through one of the insulative layers for direct contact with patient blood or other body fluids, when the sensing portion 18 (or active portion) of the analyte sensor 12 is subcutaneously placed at an insertion site. The sensing portion 18 is joined to a connection portion 24 that terminates in conductive contact pads, or the like, which are also exposed through one of the insulative layers. In alternative aspects, other types of implantable sensors, such as chemical based, optical based, or the like, may be used.

As is known in the art, the connection portion 24 and the contact pads are generally adapted for a direct wired electrical connection to a suitable monitor or sensor electronics device 100 for monitoring a user's condition in response to signals derived from the sensor electrodes 20. Further description of flexible thin film sensors of this general type may be found, e.g., in U.S. Patent No. 5,391,250, which is herein incorporated by reference. The connection portion 24 may be conveniently connected electrically to the monitor or sensor electronics device 100 or by a connector block 28 (or the like) as shown and described, e.g., in U.S. Patent No. 5,482,473, which is also herein incorporated by reference. Thus, in accordance with aspects of the present disclosure, subcutaneous sensor sets 10 may be configured or formed to work with either a wired or a wireless characteristic monitor system.

The sensor electrodes 20 may be used in a variety of sensing applications and may be configured in a variety of ways. For example, the sensor electrodes 20 may be used in physiological parameter sensing applications in which some type of biomolecule is used as a catalytic agent. For example, the sensor electrodes 20 may be used in a glucose and oxygen sensor having a glucose oxidase (GOx) enzyme catalyzing a reaction with the sensor electrodes 20. The reaction produces Gluconic Acid (C₆H₁₂O₇) and Hydrogen Peroxide (H₂O₂) in proportion to the amount of glucose present.

The sensor electrodes 20, along with a biomolecule or some other catalytic agent, may be placed in a human body in a vascular or non-vascular environment. For example, the sensor electrodes 20 and biomolecule may be placed in a vein and be subjected to a blood stream, or may be placed in a subcutaneous or peritoneal region of the human body.

The monitor 100 may also be referred to as a sensor electronics device 100. The monitor 100 may include a power source 110, a sensor interface 122, processing electronics 124, and data formatting electronics 128. The monitor 100 may be coupled to the sensor set 10 by a cable 102 through a connector that is electrically coupled to the connector block 28 of the connection portion 24. In an alternative aspect, the cable 102 may be omitted. In this aspect of the disclosure, the monitor 100 may include an appropriate connector for direct connection to the connection portion 104 of the sensor set 10. The sensor set 10 may be modified to have the connector portion 104 positioned at a different location, e.g., on top of the sensor set 10 to facilitate placement of the monitor 100 over the sensor set 10.

In aspects of the disclosure, the sensor interface 122, the processing electronics 124, and the data formatting electronics 128 are formed as separate semiconductor chips, however, alternative aspects may combine the various semiconductor chips into a single or multiple customized semiconductor chips. The sensor interface 122 connects with the cable 102 that is connected with the sensor set 10.

The power source 110 may be a battery. The battery can include three series silver oxide battery cells. In alternative aspects, different battery chemistries may be utilized, such as lithium based chemistries, alkaline batteries, nickel metalhydride, or the like, and a different number of batteries may be used. The monitor 100 provides power to the sensor set via the power source 110, through the cable 102 and cable connector 104. In an aspect of the disclosure, the power is a voltage provided to the sensor set 10. In an aspect of the disclosure, the power is a current provided to the sensor set 10. In an aspect of the disclosure, the power is a voltage provided at a specific voltage to the sensor set 10.

FIG. 2 illustrates an implantable analyte sensor and electronics for driving the implantable analyte sensor according to an aspect of the present disclosure. FIG. 2 shows a substrate or flex220 having two sides; a first side 222 which contains an electrode configuration and a second side 224 of which contains electronic circuitry. As in FIG. 2, the first side 222 of the substrate includes two counter electrode-working electrode pairs 240, 242, 244, 246 on opposite sides of a reference electrode 248. A second side 224 of the substrate includes electronic circuitry. As shown, the electronic circuitry may be enclosed in a hermetically sealed casing 226, providing a protective housing for the electronic circuitry. This allows the sensor substrate 220 to be inserted into a vascular environment or other environment which may subject the electronic circuitry to fluids. By sealing the electronic circuitry in a hermetically sealed casing 226, the electronic circuitry may operate without risk of short circuiting by the surrounding fluids. Also shown in FIG. 2, pads 228 are connected to the input and output lines of the electronic circuitry. The electronic circuitry itself may be fabricated in a variety of ways. According to an aspect of the present disclosure, the electronic circuitry may be fabricated as an integrated circuit using techniques common in the industry.

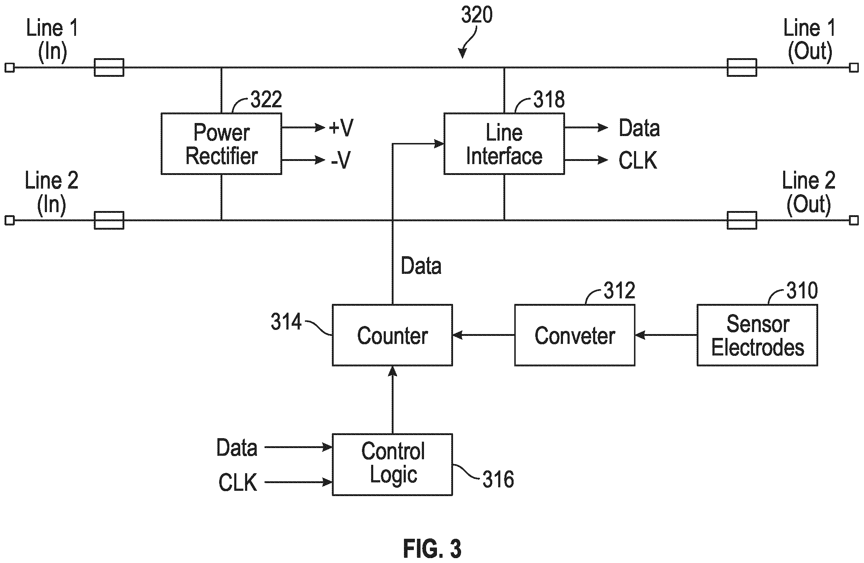

FIG. 3 illustrates a general block diagram of an electronic circuit for sensing an output of an analyte sensor according to aspects of the present disclosure. At least one pair of sensor electrodes 310 may interface to a data converter 312, the output of which may interface to a counter 314. The counter 314 may be controlled by control logic 316. The output of the counter 314 may connect to a line interface 318. The line interface 318 may be connected to input and output lines 320 and may also connect to the control logic 316. The input and output lines 320 may also be connected to a power rectifier 322.

The sensor electrodes 310 may be used in a variety of sensing applications and may be configured in a variety of ways. For example, the sensor electrodes 310 may be used in physiological parameter sensing applications in which some type of biomolecule is used as a catalytic agent. For example, the sensor electrodes 310 may be used in a glucose and oxygen sensor having a glucose oxidase (GOx) enzyme catalyzing a reaction with the sensor electrodes 310. The sensor electrodes 310, along with a biomolecule or some other catalytic agent, may be placed in a human body in a vascular or non-vascular environment. For example, the sensor electrodes 310 and biomolecule may be placed in a vein and be subjected to a blood stream.

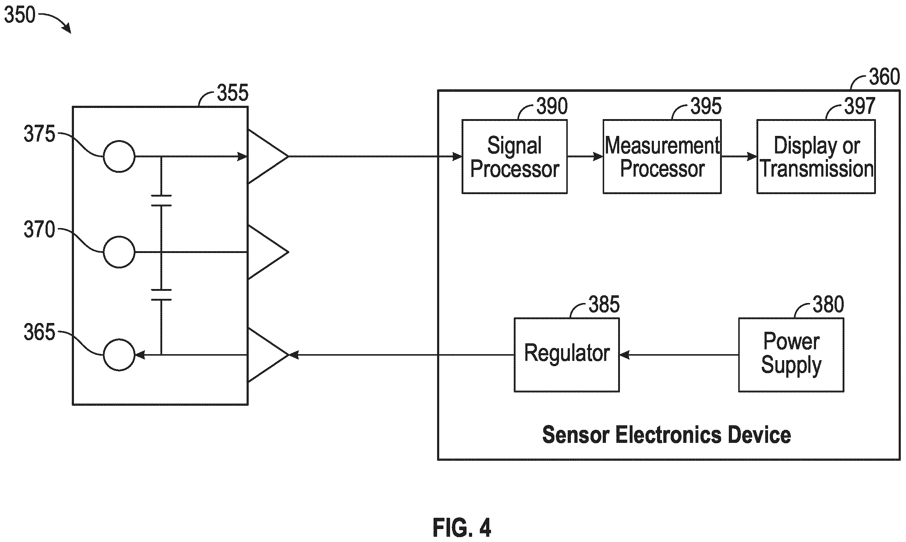

FIG. 4 illustrates a block diagram of an analyte sensor electronics device and a sensor including a plurality of electrodes according to an aspect of the disclosure. The sensor set or system 350 includes an analyte sensor 355 and a sensor electronics device 360. The analyte sensor 355 includes a counter electrode 365, a reference electrode 370, and a working electrode 375. The sensor electronics device 360 includes a power supply 380, a regulator 385, a signal processor 390, a measurement processor 395, and a display/transmission module 397. The power supply 380 provides power (in the form of either a voltage, a current, or a voltage including a current) to the regulator 385. The regulator 385 transmits a regulated voltage to the analyte sensor 355. In an aspect of the disclosure, the regulator 385 transmits a voltage to the counter electrode 365 of the analyte sensor 355.

The analyte sensor 355 creates a sensor signal indicative of a concentration of a physiological characteristic being measured. For example, the sensor signal may be indicative of a blood glucose reading. In an aspect of the disclosure, utilizing subcutaneous sensors, the sensor signal may represent a level of hydrogen peroxide in a subject. In an aspect of the disclosure, where blood or cranial sensors are utilized, the amount of oxygen is being measured by the sensor and is represented by the sensor signal. In an aspect of the disclosure, utilizing implantable or long- term sensors, the sensor signal may represent a level of oxygen in the subject. The sensor signal may be measured at the working electrode 375. In an aspect of the disclosure, the sensor signal may be a current measured at the working electrode. In an aspect of the disclosure, the sensor signal may be a voltage measured at the working electrode.

The signal processor 390 receives the sensor signal (e.g., a measured current or voltage) after the sensor signal is measured at the analyte sensor 355 (e.g., the working electrode). The signal processor 390 processes the sensor signal and generates a processed sensor signal. The measurement processor 395 receives the processed sensor signal and calibrates the processed sensor signal utilizing reference values. In an aspect of the disclosure, the reference values are stored in a reference memory and provided to the measurement processor 395. The measurement processor 395 generates sensor measurements. The sensor measurements may be stored in a measurement memory (not shown). The sensor measurements may be sent to a display/transmission device to be either displayed on a display in a housing with the sensor electronics or transmitted to an external device.

The sensor electronics device 360 may be a monitor which includes a display to display physiological characteristics readings. The sensor electronics device 360 may also be installed in a desktop computer, a pager, a television including communications capabilities, a laptop computer, a server, a network computer, a personal digital assistant (PDA), a portable telephone including computer functions, an infusion pump including a display, and/or a combination infusion pump/ analyte sensor. The sensor electronics device 360 may be housed in a cellular phone, a smartphone, a network device, a home network device, and/or other appliance connected to a home network.

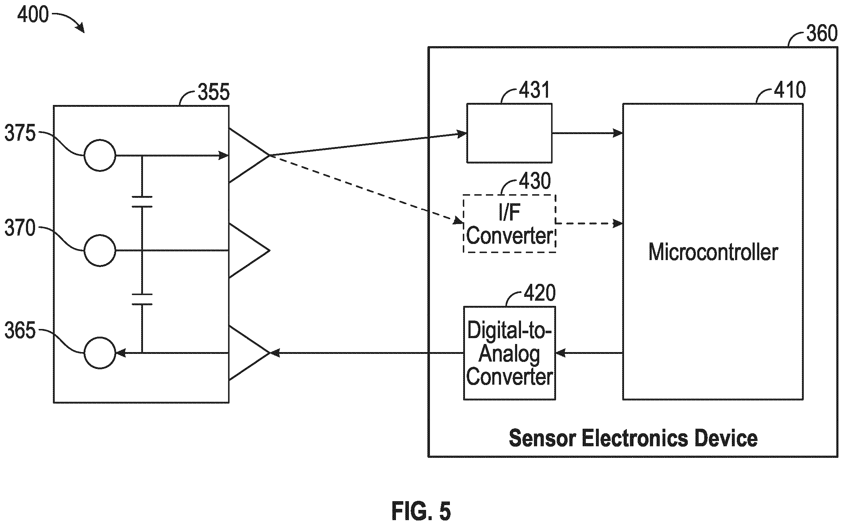

FIG. 5 illustrates an alternative aspect including an analyte sensor and a sensor electronics device according to an aspect of the present disclosure. The sensor set or sensor system 400 includes the sensor electronics device 360 and the analyte sensor 355. The analyte sensor 355 includes the counter electrode 365, the reference electrode 370, and the working electrode 375. The sensor electronics device 360 includes a microcontroller 410 and a digital-to-analog converter (DAC) 420. The sensor electronics device 360 may also include a current-to-frequency converter (I/F converter) 430.

The microcontroller 410 includes software program code or programmable logic which, when executed, causes the microcontroller 410 to transmit a signal to the DAC 420, where the signal is representative of a voltage level or value that is to be applied to the analyte sensor 355. The DAC 420 receives the signal and generates the voltage value at the level instructed by the microcontroller 410. In aspects of the disclosure, the microcontroller 410 may change the representation of the voltage level in the signal frequently or infrequently. Illustratively, the signal from the microcontroller 410 may instruct the DAC 420 to apply a first voltage value for one second and a second voltage value for two seconds.

The analyte sensor 355 may receive the voltage level or value. In an aspect of the disclosure, the counter electrode 365 may receive the output of an operational amplifier which has as inputs the reference voltage and the voltage value from the DAC 420. The application of the voltage level causes the analyte sensor 355 to create a sensor signal indicative of a concentration of a physiological characteristic being measured. In an aspect of the disclosure, the microcontroller 410 may measure the sensor signal (e.g., a current value) from the working electrode. Illustratively, a sensor signal measurement circuit 431 may measure the sensor signal. In an aspect of the disclosure, the sensor signal measurement circuit 431 may include a resistor and the current may be passed through the resistor to measure the value of the sensor signal. In an aspect of the disclosure, the sensor signal may be a current level signal and the sensor signal measurement circuit 431 may be a current-to-frequency (I/F) converter 430. The I/F converter 430 may measure the sensor signal in terms of a current reading, convert it to a frequency-based sensor signal or electrochemical impedance spectroscopy ("EIS") signal, and transmit the frequency-based sensor signal or EIS signal to the microcontroller 410. Persons skilled in the art will understand how to implement and apply EIS. Various aspects of EIS signals are described in U.S. Patent Application Publication No. US2013/0060105A1, which is hereby incorporated by reference herein in its entirety. In aspects of the disclosure, the microcontroller 410 may be able to receive frequency-based sensor signals easier than non-frequency-based sensor signals. The microcontroller 410 receives the sensor signal, whether frequency-based or non-frequency-based, and determines a value for the physiological characteristic of a subject, such as a blood glucose level. The microcontroller 410 may include program code, which when executed or run, is able to receive the sensor signal and convert the sensor signal to a physiological characteristic value.

In one aspect of the disclosure, the microcontroller 410 may convert the sensor signal to a blood glucose level. While converting the sensor signal to a blood glucose value, the microcontroller 410 may use one or more models, which are specific ways to use the sensor signal to calculate the blood glucose value. In some aspects, the microcontroller 410 may utilize measurements (e.g., sensor signals and electrochemical impedance spectroscopy (EIS) signals from the analyte sensor 355) stored within an internal memory in order to determine the blood glucose level of the subject. In some aspects, the microcontroller 410 may utilize measurements stored within a memory external to the microcontroller 410 to assist in determining the blood glucose level of the subject.

After the physiological characteristic value is determined by the microcontroller 410, the microcontroller 410 may store measurements of the physiological characteristic values for a number of time periods. For example, a blood glucose value (BG) may be sent to the microcontroller 410 from the sensor every second or five seconds, and the microcontroller may save sensor measurements for five minutes or ten minutes of BG readings. The microcontroller 410 may transfer the measurements of the physiological characteristic values to a display on the sensor electronics device 360. For example, the sensor electronics device 360 may be a monitor which includes a display that provides a blood glucose reading for a subject. In one aspect of the disclosure, the microcontroller 410 may transfer the measurements of the physiological characteristic values to an output interface of the microcontroller 410. The output interface of the microcontroller 410 may transfer the measurements of the physiological characteristic values, e.g., blood glucose values, to an external device, e.g., an infusion pump, a combined infusion pump/glucose meter, a computer, a personal digital assistant, a pager, a network appliance, a server, a cellular phone, or any computing device.

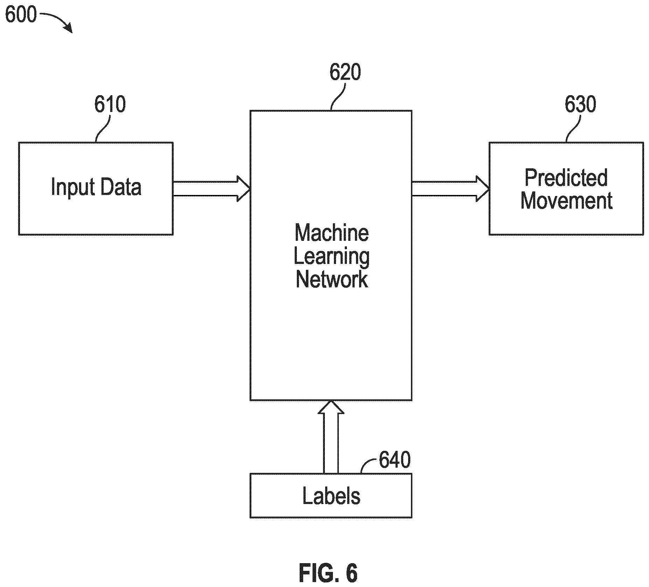

With reference to FIG. 6, a block diagram for a machine learning network 620 (e.g., a machine learning model) for classifying data in accordance with some aspects of the disclosure is shown. In some systems, a machine learning network 620 may include, for example, a convolutional neural network (CNN) and/or a recurrent neural network. A deep learning neural network includes multiple hidden layers. As explained in more detail below, the machine learning network 360 may leverage one or more classification models (e.g., CNNs, decision trees, Naive Bayes, k-nearest neighbor) to classify data. The machine learning network 620 may be executed on the measurement processor 395, a user device, and/or on a remote server. Persons of ordinary skill in the art will understand the machine learning network 620 and how to implement it.

In machine learning, a CNN is a class of artificial neural network (ANN). The convolutional aspect of a CNN relates to applying matrix processing operations to localized portions of the data, and the results of those operations (which can involve dozens of different parallel and serial calculations) are sets of many features that are delivered to the next layer. A CNN typically includes convolution layers, activation function layers, deconvolution layers (e.g., in segmentation networks), and/or pooling (typically max pooling) layers to reduce dimensionality without losing too many features. Additional information may be included in the operations that generate these features. Providing unique information that yields features that give the neural networks information can be used to provide an aggregate way to differentiate between different data that is input to the neural networks.

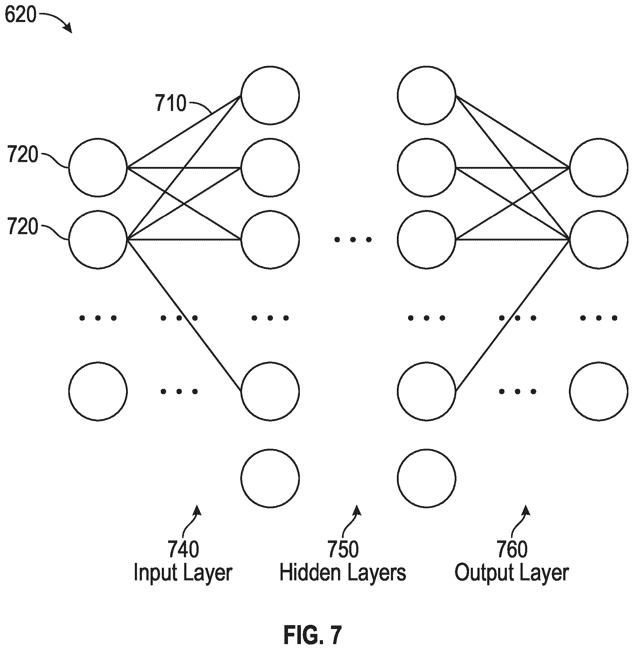

Referring to FIG. 7, generally, the machine learning network 620 (e.g., a convolutional deep learning neural network) includes at least one input layer 740, a plurality of hidden layers 750, and at least one output layer 760. The input layer 740, the plurality of hidden layers 750, and the output layer 760 all include neurons 720 (e.g., nodes). The neurons 720 between the various layers are interconnected via weights 710. Each neuron 720 in the machine learning network 620 computes an output value by applying a specific function to the input values coming from the previous layer. The function that is applied to the input values is determined by a vector of weights 710 and a bias. Learning in the deep learning neural network progresses by making iterative adjustments to these biases and weights. The vector of weights 710 and the bias are called filters (e.g., kernels) and represent particular features of the input (e.g., a particular shape). Although CNNs are used as an example, other machine learning classifiers are contemplated, for example, neighborhood component analysis (NCA), LASSO, random forest, visual cross-correlation analysis, and/or maximum relevance - minimum redundancy (MRMR), to select features of the EIS signature values. In aspects, the classification may be performed based on regression.

The machine learning network 620 may be initially trained based on feeding a dataset into the machine learning network 620 that is known to be good and/or ground truth performance data. This enables the machine learning network 620 to learn what good performance looks like and detect deviations through unsupervised learning.

The machine learning network 620 may undergo supervised learning. The machine learning network 620 may be trained based on labeling training data to optimize weights. For example, samples of sensor feature data may be taken and labeled using other sensor feature data. In some methods in accordance with this disclosure, the training may include supervised learning or semi-supervised learning. Persons of ordinary skill in the art will understand training the machine learning network 620 and how to implement it.

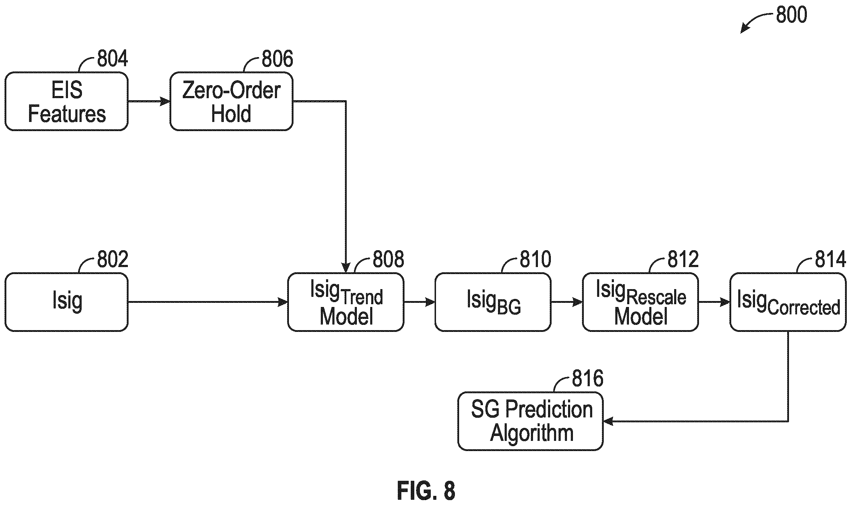

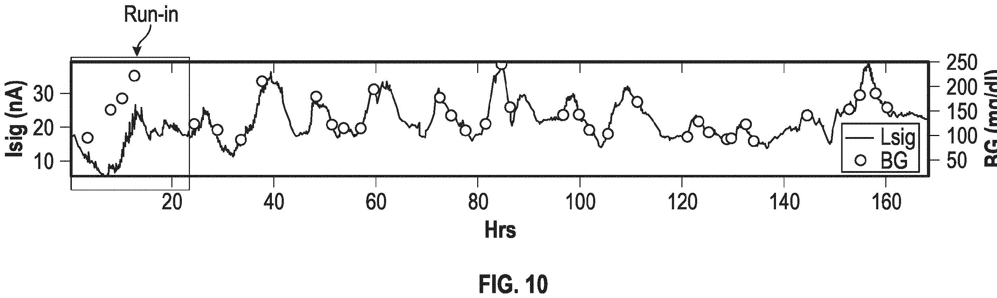

FIG. 8 is a diagram illustrating an exemplary method for adjusting real-time analyte sensor data using the machine learning network 620 of FIG. 6. The analyte sensor 355 is implanted in a body of a user and is configured to generate real-time data relating to glucose sensitivity. When the sensor is implanted into a body of a user, during day 1 the Isig values may be inaccurate (see FIG. 10). The day 1 run-in Isig values may include two components, a sensor component and the analyte signal. The baseline component may be substantially comprised of a signal contribution from factors other than the measured analyte (e.g., interfering species, non-reaction-related hydrogen peroxide, or other electroactive species with an oxidation potential that overlaps with the analyte or co-analyte). The analyte signal (e.g., glucose signal) is substantially comprised of a signal contribution from the analyte. Impedance that relates to the membrane resistance of a sensor is typically initially high and then gradually decreases as the sensor 355 is run-in.

At step 802, the Isig values of the sensor 355 are accessed. In aspects, the Isig values may be filtered, for example, by using an IIR filter and/or a FIR filter.

At step 804, EIS signature values of the sensor 355 are accessed. As described above, the EIS signature values may be computed based on an EIS signal. A microcontroller of the analyte sensor may receive an electrochemical impedance spectroscopy ("EIS") signal from one or more working electrodes of the analyte sensor and calculate real impedance (Zᵣₑₐₗ) and imaginary impedance (Z_{imag}) of the EIS signal.

At step 806, the EIS signature values may be processed using a zero-order hold. The zero-order hold is a method that is used to reconstruct signals in real-time. In zero-order hold, the continuous signal is reconstructed from its samples by holding the given sample for an interval until the next sample is received. At step 808, the EIS signature values and Isig values are input into an Isig_{trend} model. The Isig_{trend} model is a machine learning model (e.g., machine learning model 620), and may include, for example, a convolutional neural network, neighborhood component analysis (NCA), LASSO, random forest, visual cross-correlation analysis, and/or maximum relevance - minimum redundancy (MRMR), to select features of the EIS signature values. In aspects, the system 350 (FIG. 4) may select a machine learning model 620 based on criteria, such as which is the simplest machine learning model that can accurately provide results. For example, LASSO regularization may be used to identify and discard unnecessary predictors in penalized regression. In another example, NCA may be used to maximize prediction accuracy to regularized leave-one-out K-nearest neighbor classification. Visual cross-correlation analysis may be used to identify correlated features to minimize the selection of redundant predictors. The EIS signature values may be processed using feature transformation, for example, using Nyquist slopes and/or inversion, prior to being input into the machine learning model. The component values of the EIS signature may be used by the machine learning modem, for example, the real portion, the imaginary portion, the magnitude, and/or the phase of the EIS signature value.

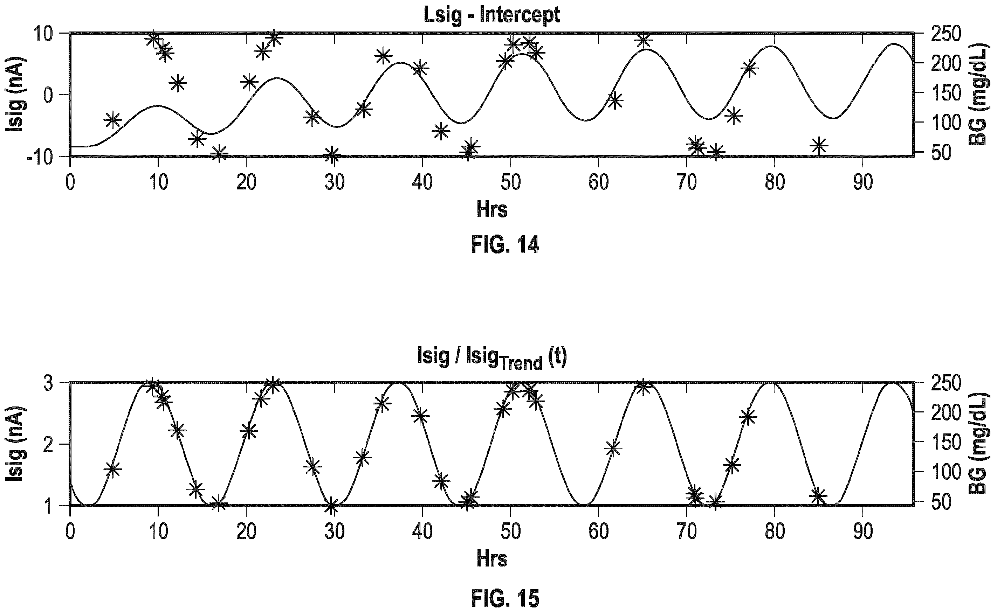

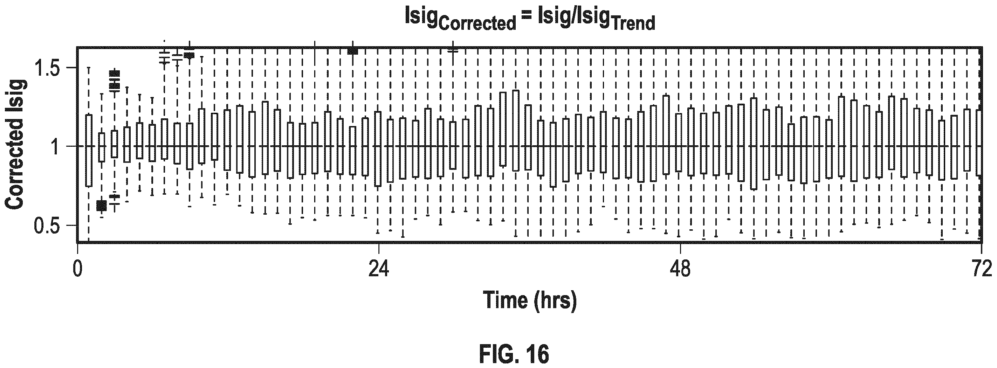

At step 810, the Isig_{trend} model estimates an Isig_{BG} value based on the EIS signature values and Isig values. In aspects, an adjusted Isig value (Isig_{corrected}) may be determined by dividing the Isig value by the Isig_{trend} value (see FIG. 15 and 16).

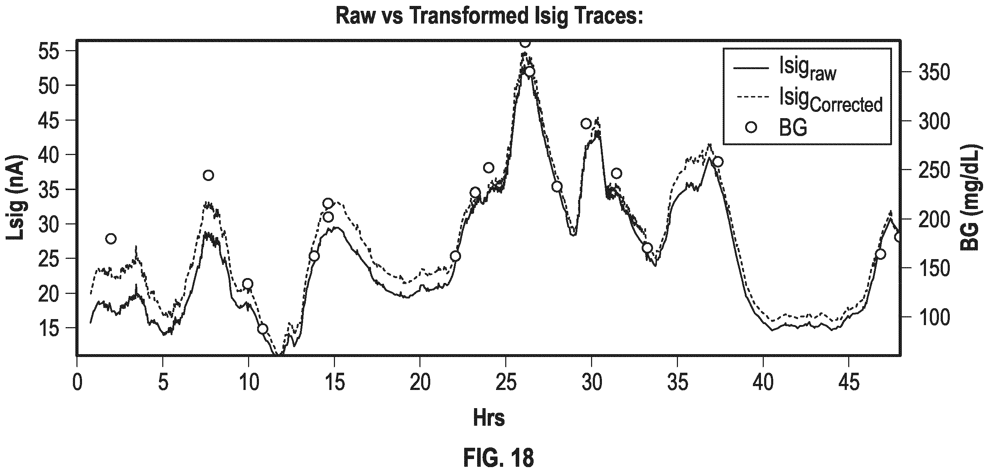

At step 812 the Isig_{BG} value is input into an Isig_{rescale} model to generate an adjusted Isig value (Isig_{corrected}) 814 (see FIG. 18). The Isig_{rescale} model may include machine learning model 620. At step 816, the adjusted Isig value is input into the sensor glucose (SG) prediction algorithm to generate a predicted SG value.

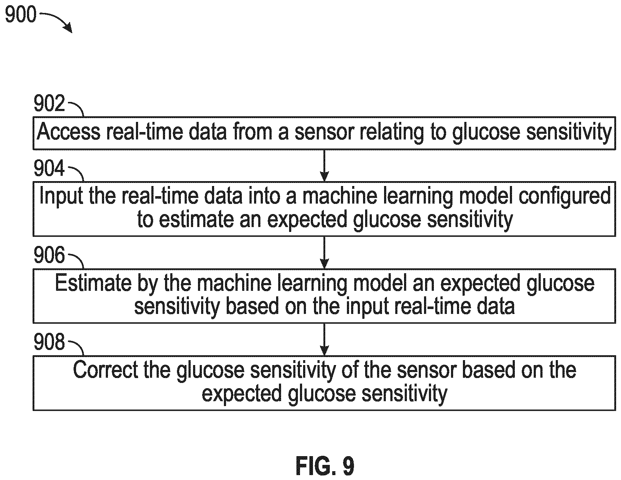

FIG. 9 illustrates a flowchart of a method 900 for adjusting real-time analyte sensor data for reducing analyte sensor 355 variability. The analyte sensor 355 is implanted in a body of a user. The analyte sensor 355 is configured to generate real-time data relating to glucose sensitivity.

At step 902, the signal processor 390 (FIG. 4) accesses real-time data from the analyte sensor 355 relating to glucose sensitivity. As noted above, during run-in, the analyte sensor SG value may be inaccurate (see FIG. 10). The real-time data may include the EIS signature values, Isig values, and/or Vcntr values. For example, real-time data may include sensor data on, for example, wear time, battery life, calibration information, electrical data, or other electrical properties of the sensor 355.

At step 904, the measurement processor 395 causes the system 350 to input the real-time data into the machine learning model 620 (FIG. 6), which estimates an expected glucose sensitivity. For example, the machine learning model 620 may analyze a trend of the real-time data and interpret a proportionality between the EIS signature values and Isig values to normalize the Isig value.

At step 906, the measurement processor 395 causes the system 350 to estimate, by the machine learning model 620, an expected glucose sensitivity based on the input real-time data.

In aspects, the machine learning model 620 may use the estimates to be fed back (e.g., active feedback) to machine learning model 620 as input to train machine learning model 620 (e.g., alone or in conjunction with user indications of the accuracy of estimates, labels associated with the inputs real-time data, or with other reference feedback information). In another aspect, machine learning model 620 may update its configurations (e.g., weights, biases, or other parameters) based on its assessment of its prediction (e.g., estimates) and reference feedback information (e.g., user indication of accuracy, reference labels, or other information). In another aspect, where machine learning model 620 is a neural network, connection weights may be adjusted to reconcile differences between the neural network's prediction and the reference feedback. In a further aspect, one or more neurons (or nodes) of the neural network may require that their respective errors are sent backward through the neural network to them to facilitate the update process (e.g., backpropagation of error). Updates to the connection weights may, for example, be reflective of the magnitude of error propagated backward after a forward pass has been completed. In this way, for example, the machine learning model 620 may be trained to generate better estimates.

In aspects, estimating, by the machine learning model 620, the compensated glucose sensitivity includes estimating an Isig trend over time for the sensor 355. The Isig trend over time is independent of any sensed glucose fluctuations. In aspects, the measurement processor 395 causes the system 350 to stabilize the calibration ratio based on correcting Isig using the estimated Isig trend over time and the EIS signature value. A calibration ratio may include a blood glucose value divided by Isig.

The real-time data includes a portion that is based on a blood glucose value and a portion that is unrelated to the blood glucose value. In aspects, the measurement processor 395 causes the system 350 to determine, by the machine learning model, a portion of the real-time data that is unrelated to the blood glucose value and generate an adjusted Isig based on the determined portion of the real-time data that is unrelated to the blood glucose value.

At step 908, the measurement processor 395 causes the system 350 to correct the glucose sensitivity of the sensor 355 based on the expected glucose sensitivity. The corrected glucose sensitivity may then be used by the measurement processor 395 to provide a more accurate SG reading in real-time.

The disclosed technology provides the benefit of reducing the run-in period of the sensor and reducing sensor variability. Although initial day 1 run-in is used as an example, the disclosed technology may be used to improve sensor lifetime, since similar sensor behavior of decreased sensitivity and current-impedance proportionality may be observed in late wear performance and since sensitivity loss represents a main contributor to early sensor termination. For example, as wear time of a sensor device increases, the sensitivity of the sensor may decrease. The acceptable range of glucose sensitivity may therefore change dynamically with wear time (or another sensor electrical property). In some embodiments, glucose sensitivity may vary as sensor features change, for example, due to variability in the sensing environment, physiological dynamics, or sensor manufacturing. The mosaic of models described herein captures these dynamic changes and outputs a more accurate glucose sensitivity.

FIG. 10 is a graph that illustrates day 1 run-in for an implanted sensor. The graph shows that approximately during the first twenty four hours the current read from the sensor needs time to stabilize for a more accurate reading.

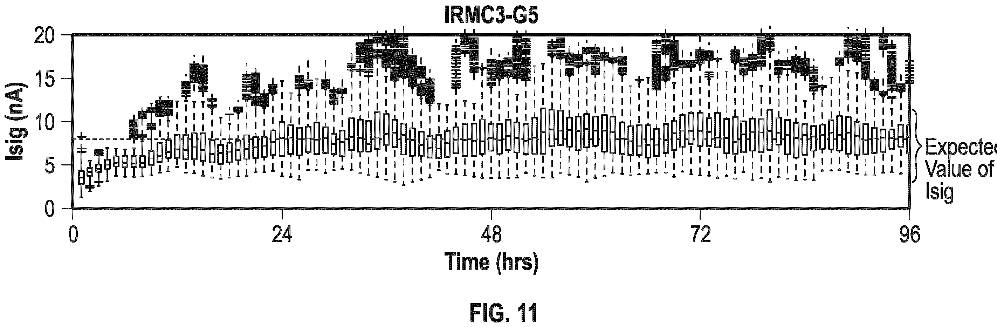

FIG. 11 illustrates example measured Isig values vs expected Isig values over time. As shown in the graph, Isig values initially start at about 4nA and increase over the first twenty four hours or so.

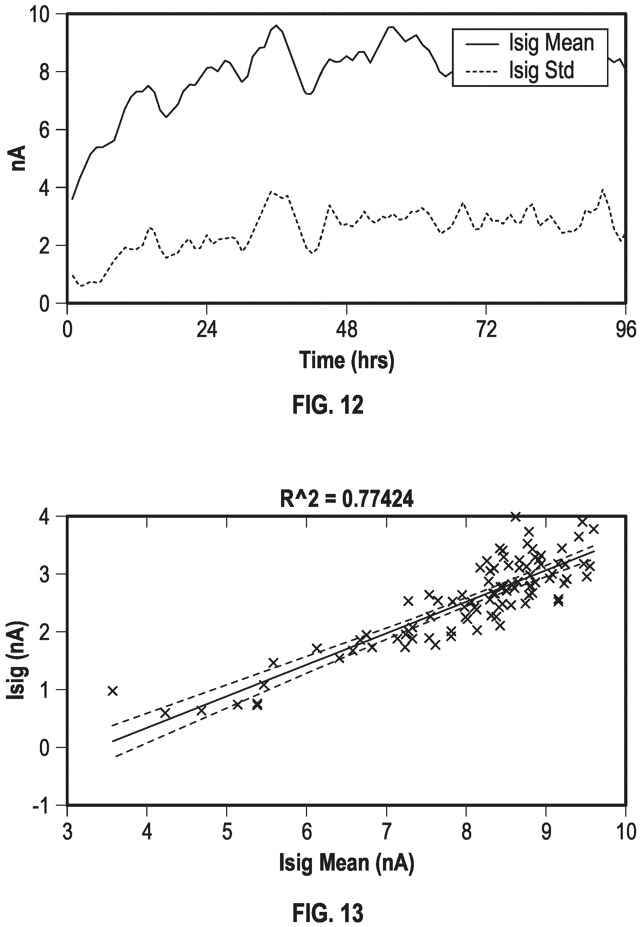

FIG. 12 is a graph illustrating example Isig mean and Isig standard deviation over time. As noted in the graph, the Isig mean value starts at about 4nA for the first few hours and rises to a mean value of about 10 nA at the end of the first twenty four hours.

FIG. 13 is a graph illustrating R² for an example Isig mean vs Isig standard deviation. In the graph, the Isig standard deviation vs Isig mean have a R² value of about 77%. An R² value shows how well a model predicts an outcome of a dependent variable.

FIG. 14 is a graph illustrating an example of Isig and blood glucose (BG) in mg/dl over time for a sensor that is not adjusted.

FIG. 15 is a graph illustrating an example of adjusted Isig and blood glucose (BG) over time. In this graph, Isig has been adjusted using the disclosed technology. As is shown by the graph, the first twenty four hours of adjusted Isig values track the rest of the time period.

FIG. 16 is a graph illustrating an example of adjusted Isig (Isig_{corrected}) and expected Isig values over time over time. In this graph, Isig has been adjusted using the disclosed methods.

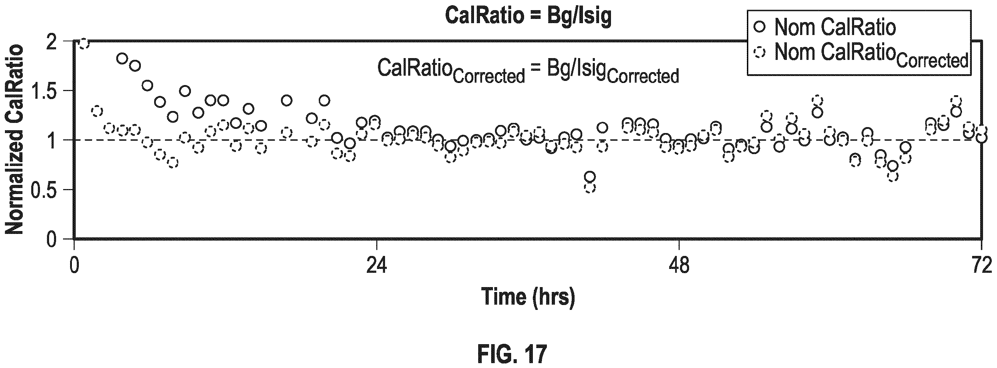

FIG. 17 is a graph illustrating an example of a normalized calibration ratio for both the original measured Isig and the adjusted Isig. The term "calibration ratio" as used herein is the BG/Isig. As can be seen in the graph, the calibration ratio for the adjusted Isig is flatter than the calibration ratio for the original measured Isig.

FIG. 18 is a graph illustrating example raw Isig values, BG, and adjusted Isig values. As is shown in the graph, the adjusted Isig values better track the actual BG values than the original measured Isig values.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, the above-described servers and computing devices.

While the description above refers to particular aspects of the present disclosure, it will be understood that many modifications may be made without departing from the spirit thereof. Additional steps and changes to the order of the algorithms can be made while still performing the key teachings of the present disclosure. Thus, the accompanying claims are intended to cover such modifications as would fall within the true scope and spirit of the present disclosure. The presently disclosed aspects are, therefore, to be considered in all respects as illustrative and not restrictive, the scope of the disclosure being indicated by the appended claims rather than the foregoing description. Unless the context indicates otherwise, any aspect disclosed herein may be combined with any other aspect or aspects disclosed herein. All changes that come within the meaning of, and range of, equivalency of the claims are intended to be embraced therein.

Further disclosed herein is the subject-matter of the following clauses:
1. A system for reducing analyte sensor variability, the system comprising:
   a sensor configured to generate real-time data relating to glucose sensitivity; one or more processors; and
   one or more processor-readable media storing instructions which, when executed by the one or more processors, causes performance of
      accessing the real-time data from the sensor relating to glucose sensitivity;
      inputting the real-time data into a machine learning model;
      estimating by the machine learning model an expected glucose sensitivity based on the real-time data; and
      correcting the glucose sensitivity of the sensor based on the expected glucose sensitivity.
2. The system of clause 1, wherein the real-time data includes electrical properties associated with the sensor.
3. The system of clause 1 or 2, wherein the real-time data includes an Isig value and at least one of a Vcntr value or an EIS signature value.
4. The system of clause 3 or of one of clauses 1 to 3, wherein estimating by the machine learning model the corrected glucose sensitivity includes estimating an Isig trend over time for the sensor.
5. The system of clause 4 or of one of clauses 1 to 4, wherein the Isig trend over time is independent of any sensed glucose fluctuations.
6. The system of clause 4 or of one of clauses 1 to 5, wherein a calibration ratio is a blood glucose value divided by Isig, and the instructions, when executed by the one or more processors, further causes performance of
   stabilizing the calibration ratio based on correcting Isig using the estimated Isig trend over time and the EIS signature value.
7. The system of clause 4 or of one of clauses 1 to 6, wherein the real-time data includes a portion that is based on a blood glucose value and a portion that is unrelated to the blood glucose value, and the instructions, when executed by the one or more processors, further causes performance of
   determining, by the machine learning model, a portion of the real-time data that is unrelated to the blood glucose value.
8. The system of clause 7 or of one of clauses 1 to 7, wherein the instructions, when executed by the one or more processors, further causes performance of
   generating an adjusted Isig based on the determined portion of the real-time data that is unrelated to the blood glucose value.
9. The system of clause 1 or of one of clauses 1 to 8, wherein when correcting the glucose sensitivity, the instructions, when executed by the one or more processors, further causes performance of
   estimating by the machine learning model a calibration factor as a function of time based on the real-time data; and
   calibrating the real-time data at least in part based on the calibration factor.
10. The system of clause 7 or of one of clauses 1 to 9, wherein the portion of the real-time data that is unrelated to the blood glucose value is based on a design configuration of the sensor.
11. A processor-implemented method of correcting real-time sensor data, the method comprising:
   accessing real-time data from a sensor relating to glucose sensitivity;
   inputting the real-time data into a machine learning model;
   estimating by the machine learning model an expected glucose sensitivity based on the input real-time data; and
   correcting the glucose sensitivity based on the expected glucose sensitivity.
12. The processor-implemented method of clause 11, wherein the real-time data includes electrical properties associated with the sensor.
13. The processor-implemented method of clause 11 or 12, wherein the real-time data includes an Isig value and at least one of a Vcntr value or an EIS signature value.
14. The processor-implemented method of clause 13 or of one of clauses 11 to 13, wherein estimating by the machine learning model the corrected glucose sensitivity includes estimating an Isig trend over time for the sensor.
15. The processor-implemented method of clause 14 or of one of clauses 11 to 14, wherein the Isig trend over time is independent of any sensed glucose fluctuations.
16. The processor-implemented method of clause 14 or of one of clauses 11 to 15, wherein a calibration ratio is a blood glucose value divided by Isig, and the method further comprises stabilizing the calibration ratio over time based on correcting Isig using the estimated Isig trend over time.
17. The processor-implemented method of clause 14 or of one of clauses 11 to 16, wherein the real-time data includes a portion that is based on a blood glucose value and a portion that is unrelated to the blood glucose value, and the method further comprises determining, by the machine learning model, a portion of the real-time data that is unrelated to the blood glucose value.
18. The processor-implemented method of clause 17 or of one of clauses 11 to 17, wherein the method further comprises generating an adjusted Isig based on the determined portion of the real-time data that is unrelated to the blood glucose value.
19. The processor-implemented method of clause 11 or of one of clauses 11 to 18, further comprising:
   estimating by the machine learning model a calibration factor as a function of time based on the input real-time data; and
   calibrating the real-time data at least in part based on the calibration factor.
20. One or more non-transitory processor readable media storing instructions which, when executed by one or more processors, cause performance of
   accessing real-time data from a sensor relating to glucose sensitivity;
   inputting the real-time data into a machine learning model;
   estimating by the machine learning model an expected glucose sensitivity based on the input real-time data; and
   correcting the glucose sensitivity based on the expected glucose sensitivity.

## Claims

1. A system for reducing analyte sensor variability, the system comprising:
a sensor configured to generate real-time data relating to glucose sensitivity;
one or more processors; and
one or more processor-readable media storing instructions which, when executed by the one or more processors, causes performance of
accessing the real-time data from the sensor relating to glucose sensitivity;
inputting the real-time data into a machine learning model;
estimating by the machine learning model an expected glucose sensitivity based on the real-time data; and
correcting the glucose sensitivity of the sensor based on the expected glucose sensitivity.

2. The system of claim 1, wherein the real-time data includes electrical properties associated with the sensor.

3. The system of claim 1 or 2, wherein the real-time data includes an Isig value and at least one of a Vcntr value or an EIS signature value.

4. The system of one of claims 1 to 3, wherein estimating by the machine learning model the corrected glucose sensitivity includes estimating an Isig trend over time for the sensor, particularly
wherein the Isig trend over time is independent of any sensed glucose fluctuations.

5. The system of claim 4, wherein a calibration ratio is a blood glucose value divided by Isig, and the instructions, when executed by the one or more processors, further causes performance of
stabilizing the calibration ratio based on correcting Isig using the estimated Isig trend over time and the EIS signature value.

6. The system of one of claims 1 to 5, wherein the real-time data includes a portion that is based on a blood glucose value and a portion that is unrelated to the blood glucose value, and the instructions, when executed by the one or more processors, further causes performance of
determining, by the machine learning model, a portion of the real-time data that is unrelated to the blood glucose value,
particularly
wherein the portion of the real-time data that is unrelated to the blood glucose value is based on a design configuration of the sensor.

7. The system of claim 6, wherein the instructions, when executed by the one or more processors, further causes performance of
generating an adjusted Isig based on the determined portion of the real-time data that is unrelated to the blood glucose value.

8. The system of one of claims 1 to 7, wherein when correcting the glucose sensitivity, the instructions, when executed by the one or more processors, further causes performance of
estimating by the machine learning model a calibration factor as a function of time based on the real-time data; and
calibrating the real-time data at least in part based on the calibration factor.

9. A processor-implemented method of correcting real-time sensor data, the method comprising:
accessing real-time data from a sensor relating to glucose sensitivity;
inputting the real-time data into a machine learning model;
estimating by the machine learning model an expected glucose sensitivity based on the input real-time data; and
correcting the glucose sensitivity based on the expected glucose sensitivity.

10. The processor-implemented method of claim 9, wherein the real-time data includes electrical properties associated with the sensor, and/or
wherein the real-time data includes an Isig value and at least one of a Vcntr value or an EIS signature value.

11. The processor-implemented method of claim 9 or 10, wherein estimating by the machine learning model the corrected glucose sensitivity includes estimating an Isig trend over time for the sensor, particularly
wherein the Isig trend over time is independent of any sensed glucose fluctuations.

12. The processor-implemented method of claim 11, wherein a calibration ratio is a blood glucose value divided by Isig, and the method further comprises stabilizing the calibration ratio over time based on correcting Isig using the estimated Isig trend over time.

13. The processor-implemented method of one of claims 9 to 12, wherein the real-time data includes a portion that is based on a blood glucose value and a portion that is unrelated to the blood glucose value, and the method further comprises determining, by the machine learning model, a portion of the real-time data that is unrelated to the blood glucose value, particularly wherein the method further comprises generating an adjusted Isig based on the determined portion of the real-time data that is unrelated to the blood glucose value.

14. The processor-implemented method of one of claims 9 to 13, further comprising:
estimating by the machine learning model a calibration factor as a function of time based on the input real-time data; and
calibrating the real-time data at least in part based on the calibration factor.

15. One or more non-transitory processor readable media storing instructions which, when executed by one or more processors, cause performance of
accessing real-time data from a sensor relating to glucose sensitivity;
inputting the real-time data into a machine learning model;
estimating by the machine learning model an expected glucose sensitivity based on the input real-time data; and
correcting the glucose sensitivity based on the expected glucose sensitivity.
